# EUROPEAN PATENT APPLICATION

(11) **EP 4 785 967 A1**
(43) Date of publication of application: **05.08.2026**
(21) Application number: 25778223.5
(22) Date of filing: 11.02.2025
(51) Int. Cl.: A61L 24/04, A61L 24/08, A61L 24/10, A61L 24/00

(54) **DEGRADABLE IN-VIVO PRESSURE-SENSITIVE ADHESIVE TAKING DECELLULARIZED MATRIX MATERIAL AS BASE MATERIAL AND PREPARATION METHOD THEREFOR**

(30) Priority: 26.03.2024 CN 202410346123
(71) Applicant: Shanghai Pollagen Medical Materials Co., Ltd, Shanghai 200120 (CN)
(72) Inventor: WU, Weidong, Shanghai 201203 (CN); NIU, Wenya, Shanghai 201203 (CN); LI, Xiaomeng, Shanghai 201203 (CN); LI, Chunming, Shanghai 201203 (CN)
(74) Representative: Rapisardi, Mariacristina
(86) International application number: PCT/CN2025/076800
(87) International publication number: WO 2025/200807

(57) **Abstract**

The present application provides a degradable in-vivo pressure-sensitive adhesive taking a decellularized matrix material as a base material and a preparation method therefor. The degradable in-vivo pressure-sensitive adhesive comprises a biological base material layer and pressure-sensitive adhesive layers arranged on both sides of the biological base material layer. On the basis of the total mass of raw materials of the pressure-sensitive adhesive layer as 100%, the raw materials of the pressure-sensitive adhesive layer comprise, by mass percentage, 5%-18% of a tissue-active material, 5%-25% of a base material, 10%-35% of a thickener, 10%-50% of a plasticizer, and 10%-60% of deionized water. The degradable in-vivo pressure-sensitive adhesive provided by the present application not only has good compatibility with human tissues, but also has adjustable viscosity. Moreover, the degradable in-vivo pressure-sensitive adhesive can be absorbed or completely discharged out of the body after degradation in vivo. Therefore, it is suitable for in-vivo wound closure, prevention of fluid (gas) leakage at suture sites, tissue repair, drug delivery, wound healing, and other applications.

## Description

### TECHNICAL FIELD

The present application belongs to the technical field of pressure-sensitive adhesive materials and, in particular, relates to a degradable pressure-sensitive adhesive for use *in vivo* using a decellularized extracellular matrix (dECM) material as a substrate and a method for its preparation.

### BACKGROUND

Pressure-sensitive adhesive is an adhesive that is sensitive to pressure. This type of adhesive is subjected to adhesion when pressure is applied, while the pressure-sensitive adhesive is not subjected to adhesion when no pressure is applied. Therefore, existing bioadhesive can be replaced with the pressure-sensitive adhesive for use. The pressure-sensitive adhesive has attracted attention due to convenient use and the ability to quickly fix and adhere to the required item. Especially in the medical field, medical pressure-sensitive adhesives are generally used for exposed areas such as the suture of epidermal wounds or the securing of bandages, thereby reducing the risk of infection caused by non-degradable residues of traditional adhesive tapes on wounds.

However, at present, the medical pressure-sensitive adhesive disclosed in the existing art cannot be used inside the body, and the internal adhesive applied is limited to the sealants such as ProgelTM, BioGlue^{®}, Tisseel and Adherus. The sealant seals gaps on the surface of tissues such as the lung or the dura mater to meet the need under a particular surgery. Clinically, according to different needs of internal adhesion sites, the applicable scenarios are also different, such as the closure of wounds, the prevention of liquid (gas) leakage from sutures, the repair of tissues, the delivery of drugs, the healing of wounds and other uses. However, the sealant currently used cannot implement the multiple uses described above.

To solve the above problems, researchers have developed a degradable pressure-sensitive adhesive. This adhesive enables similar or dissimilar surfaces to be connected to each other, thereby providing a new idea for the development of internal adhesion materials applicable to different scenarios. For example, CN113908327A discloses that a molecular weight of a block polyester and a proportion of a multi-component block polyester are adjusted, the block polyester is melted into a film after a series of complex and cumbersome processes, this film is coated with an adhesive and covered on polytetrafluoroethylene to form a bioadhesive double-sided adhesive. The raw materials used in this adhesive are mostly degradable materials synthesized after production processes such as chemical modification. However, the degradation products of these degradable materials are prone to the problems such as inflammatory responses after concentrated release.

In addition, CN115895593A discloses a biodegradable pressure-sensitive adhesive. In this biodegradable pressure-sensitive adhesive, a ratio of a hard monomer to a soft monomer is adjusted so that a glass transition temperature of the entire pressure-sensitive adhesive can be adjusted. The biodegradable pressure-sensitive adhesive tape prepared from this adhesive has advantages including large initial tack, high peel strength, good holding power and good biodegradability. However, the application of the biodegradable pressure-sensitive adhesive tape to the human body is not involved.

Therefore, in this field, there is an urgent need to develop a degradable pressure-sensitive adhesive that is not only applicable to various application scenarios but also does not generate any rejection response with a simple and easy-to-implement preparation method.

### SUMMARY

The present application provides a degradable pressure-sensitive adhesive for use *in vivo* using a dECM material as a substrate and a method for its preparation. The degradable pressure-sensitive adhesive for use *in vivo* provided in the present application has good compatibility with human tissues and adjustable viscosity. Moreover, this adhesive for the use *in vivo* can also be absorbed or completely excreted *in vitro* after *in vivo* degradation. Therefore, the in vivo degradable pressure-sensitive adhesive is applicable to the closure of wounds, the prevention of liquid (gas) leakage from sutures, tissue repair, wound healing and other uses *in vivo*.

In a first aspect, the present application provides a degradable pressure-sensitive adhesive for use *in vivo* using a dECM material as a substrate. The degradable pressure-sensitive adhesive for use *in vivo* includes a biological substrate layer and a pressure-sensitive adhesive layer disposed on two sides of the biological substrate layer.

Based on a total mass of 100% for a raw material of the pressure-sensitive adhesive layer, the raw material of the pressure-sensitive adhesive layer includes a tissue active material with a mass percentage content of 5% to 18%, a base material with a mass percentage content of 5% to 25%, a thickener with a mass percentage content of 10% to 35%, a plasticizer with a mass percentage content of 10% to 50% and deionized water with a mass percentage content of 10% to 60%.

The present application provides a degradable biological pressure-sensitive adhesive for a use *in vivo*. On the one hand, a biomaterial is used as the substrate layer and has excellent biocompatibility. Therefore, body reactions such as rejection and inflammation do not occur after the biomaterial is applied. Degradation products of some degradable materials synthesized after production processes of physical or chemical modification disclosed in the existing art, such as bioadhesives using caprolactone, glycolide or lactide as the substrate, are prone to the problems such as inflammatory responses after concentrated release. On the other hand, the adhesive of the in vivo degradable pressure-sensitive adhesive is obtained without the need for complex chemical modification and process synthesis, and the biologically derived tissue active ingredient is added to some of the pressure-sensitive adhesive, which not only provides the pressure-sensitive adhesive good fluidity and water retention, but also promotes the healing of wounds and the repair of defective tissues. Moreover, other components of the pressure-sensitive adhesive are also degradable raw materials *in vivo* with excellent biocompatibility and high safety for the use *in vivo*, thereby avoiding problems such as inflammatory responses caused by adhesives prepared through means such as chemical synthesis and modification.

The viscosity of the degradable pressure-sensitive adhesive for use *in vivo* provided in the present application can be adjusted to meet pasting requirements of different scenarios. For example, for scenarios such as hemostasis, sealing and reducing body fluid exchange and infectious contamination between regions, a degradable medical pressure-sensitive adhesive with a relatively large viscosity can be applied, while for some scenarios that only need temporary bonding, such as a scenario of transferring patch materials *in vitro* to tissues and organs, a degradable medical pressure-sensitive adhesive with a relatively small viscosity can be applied. In addition, the above pressure-sensitive adhesive can be completely degraded after the implantation into the body, no foreign matter remains, and no encapsulation tissue or hard lump tissue is formed. In particular, when the image detection is performed again after a cancerous tissue is removed, no shadow structure is generated. Optionally, a pharmaceutical ingredient may also be added to the component of the pressure-sensitive adhesive. The pressure-sensitive adhesive is attached to the skin or the tissue during application, and the drug is gradually released with the occurrence of a degradation reaction to implement a function of drug delivery.

In the present application, the tissue active material has a mass percentage content of 5% to 18%, which may be, for example, 5%, 8%, 10%, 12%, 15% or 18%.

In the present application, the mass percentage content of the tissue active material is adjusted so that the degradable pressure-sensitive adhesive for use *in vivo* can have a function of repairing tissues or wounds. If the content is too low, a good repair effect cannot be achieved. If the content is too high, the viscosity of the degradable pressure-sensitive adhesive for use *in vivo* decreases after irradiation sterilization.

In the present application, the base material has a mass percentage content of 5% to 25%, which may be, for example, 5%, 8%, 10%, 12%, 15%, 18%, 20%, 22% or 25%.

In the present application, the mass percentage content of the base material is adjusted so that the degradable pressure-sensitive adhesive for use *in vivo* can have a good bonding property. Since the base material is a component that plays a major role in bonding two adherends together, the base material determines the basic performance of the adhesive. If the content is too low, the viscosity is low, making the adhesive inapplicable to some application scenarios with requirements for relatively high strength. An excessively high content may cause an excessively high viscosity of the adhesive, making the adhesive difficult to effectively coat onto surfaces that need to be bonded and affecting the fluidity and coating performance.

In the present application, the addition of the base material provides a good bonding property to the pressure-sensitive adhesive.

In the present application, the thickener has a mass percentage content of 10% to 35%, which may be, for example, 10%, 15%, 20%, 22%, 25%, 30% or 35%.

In the present application, the mass percentage content, viscosity and fluidity of the thickener are adjusted to meet application requirements. If the content is too low, the adhesive may be too fluid, making the adhesive difficult to control and coat. This may cause an excessively thin adhesive during application, thereby affecting the adhesion performance of the adhesive. On the contrary, the viscosity of the adhesive is significantly increased, making the adhesive too viscous. This may cause the adhesive difficult to effectively coat and adhere during application.

In the present application, the addition of the thickener can provide a good viscosity to the pressure-sensitive adhesive to meet requirements of particular surgical situations such as the closure of wounds, the repair of tissues, the delivery of drugs, the reinforcement of tissues, wound healing or other uses.

In the present application, the plasticizer has a mass percentage content of 10% to 50%, which may be, for example, 10%, 17%, 20%, 22%, 27%, 30%, 32%, 37%, 40%, 42%, 45%, 48% or 50%.

In the present application, the flexibility of the adhesive is adjusted through the adjustment of the mass percentage content of the plasticizer, making the adhesive more adaptable to and resistant to the deformation, vibration or temperature changes of surfaces. An excessively low content may cause insufficient adhesion, making the adhesive difficult to adaptable to objects with different shapes, structures or surface properties. On the contrary, a plasticizer with an excessively high content may make the adhesive too viscous, resulting in poor fluidity of the adhesive. This may affect the uniformity of the coating and application.

In the present application, the addition of the plasticizer can provide the flexibility, ductility and plasticity to the pressure-sensitive adhesive. These effects make the adhesive easier to form elastic and soft connections on different surfaces and improve the applicability of the adhesive.

In the present application, the deionized water has a mass percentage content of 10% to 60%, which may be, for example, 10%, 20%, 30%, 40%, 50% or 60%.

Further preferably, based on a total mass of 100% for the pressure-sensitive adhesive layer, the raw materials of the pressure-sensitive adhesive layer includes a base material with a mass percentage content of 8% to 15%, a tissue active material with a mass percentage content of 10% to 16%, a thickener with a mass percentage content of 20% to 30%, a plasticizer with a mass percentage content of 15% to 20% and deionized water with a mass percentage content of 19% to 45%.

Preferably, the tissue active material includes any one of a skin, a pericardium, an amniotic membrane, a small intestinal submucosa or a bladder basement membrane of a mammal or a combination at least two of dECM gels.

The dECM gel is prepared by decellularizing the above biomaterial, followed by digesting the biomaterial. Existing methods can be used for the preparation. For a specific decellularization method, reference may be made to a method for preparing extracellular matrix mentioned in paragraphs [0076-0082] of CN115006597B. For a specific digestion method for preparing the dECM gel, reference may be made to a method for preparing collagen gels mentioned in paragraphs [0091-0093] of the same patent.

Preferably, the base material includes a degradable natural macromolecular compound and/or a degradable modified natural macromolecular compound.

Preferably, the degradable natural macromolecular compound includes any one or a combination of at least two of starch, dextrin, peach gum, arabic gum, bone glue, hide glue, gelatin, fish glue, shellac, vegetable protein or casein.

Preferably, the degradable modified natural macromolecular compound includes any one or a combination of at least two of carboxymethyl cellulose, modified starch or polyvinyl alcohol.

Preferably, the thickener includes any one or a combination of at least two of starch, modified starch, xanthan gum, maltitol, fructooligosaccharide, sorbitol, xylitol, lactitol, mannitol, erythritol, hydrogenated starch hydrolysate, gelatin, cellulose, methyl cellulose, hydroxyethyl cellulose, hydroxypropyl methylcellulose, sodium carboxymethyl cellulose, arabic gum, tamarind gum, sesbania gum, agar, sodium alginate, carrageenan, pectin or β-cyclodextrin.

Preferably, the plasticizer includes a polyol compound.

Preferably, the polyol compound includes any one or a combination of at least two of glycerol, sorbitol or ethylene glycol.

Preferably, the base material, the thickener and the plasticizer are of different types.

Further preferably, the base material includes carboxymethyl cellulose. The degree of substitution of the carboxymethyl cellulose ranges from 0.59 to 1.0 and may be, for example, 0.59, 0.6, 0.7, 0.8, 0.9 or 1.0. The viscosity of the carboxymethyl cellulose ranges from 600 to 1000 mpa·s and may be, for example, 600 mpa·s, 650 mpa·s, 700 mpa·s, 750 mpa·s, 800 mpa·s, 850 mpa·s, 900 mpa·s, 950 mpa·s or 1000 mpa·s.

Further preferably, the thickener includes arabic gum.

Further preferably, the plasticizer includes glycerol.

Preferably, based on the total mass of 100% for the raw material of the pressure-sensitive adhesive layer, the raw material of the pressure-sensitive adhesive layer further includes 0.1% to 21.6% of a humectant and 2.5% to 5.2% of an antioxidant.

In the present application, the humectant has a mass percentage content of 0.1% to 21.6%, which may be, for example, 0.1%, 0.5%, 1%, 2.5%, 5%, 8%, 10%, 12%, 15%, 18%, 20%, 21% or 21.6%.

In the present application, adjusting the mass percentage content of the humectant can prevent the pressure-sensitive adhesive from drying and curing in a container, thereby ensuring that the pressure-sensitive adhesive maintains effects of good fluidity and application performance during storage and use. If the content is too low, the pressure-sensitive adhesive cannot be prevented from drying and curing. If the content is too high, the viscosity of the pressure-sensitive adhesive is changed, and the weather resistance of the pressure-sensitive adhesive is highly likely to be affected. For example, the product is affected in a high-humidity environment.

In the present application, the antioxidant has a mass percentage content of 2.5% to 5.2%, which may be, for example, 2.5%, 2.8%, 3%, 3.5%, 4%, 4.5%, 5% or 5.2%.

In the present application, adjusting the mass percentage content of the antioxidant can reduce or prevent the occurrence of the color change of the pressure-sensitive adhesive due to oxidation. This is very important for the application that needs appearance consistency. If the content is too low, the pressure-sensitive adhesive cannot be prevented from oxidation and discoloration. An excessively high content may cause an unstable product, affect the performance of the product during storage and increase the production cost.

Preferably, the humectant includes a polyol humectant and/or a sugar humectant.

In the present application, the humectant may further include an analog of natural moisturizing factor, which may be, for example, any one or a combination of at least two of sodium lactate or trehalose.

Preferably, the antioxidant includes any one or a combination of at least two of vitamin E, vitamin C, flavonoid, tea polyphenol, phytic acid, glucosamine, lactonic acid or sodium hyaluronate.

In the present application, the pressure-sensitive adhesive layer has a thickness of 0.2-2 mm, which may be, for example, 0.2 mm, 0.5 mm, 0.8 mm, 1 mm, 1.2 mm, 1.5 mm, 1.8 mm or 2 mm.

In the present application, adjusting the thickness of the pressure-sensitive adhesive layer can selectively adjust the viscosity of the pressure-sensitive adhesive. A relatively thin coating may cause insufficient adhesion which cannot meet usage requirement. A relatively thick coating may cause increased internal stress, affect the stability and durability of the pressure-sensitive adhesive and increase the production cost.

Preferably, a material of the biological substrate layer includes the dECM material.

Preferably, the dECM material includes any one or a combination of at least two of them: skin, pericardium, amniotic membrane, small intestinal submucosa or bladder basement membrane of a mammal.

For a specific decellularization method, an existing method can be used for the preparation. For example, reference may be made to the method for preparing extracellular matrix mentioned in paragraphs [0076-0082] of CN115006597B.

In a second aspect, the present application provides a method for preparing the degradable pressure-sensitive adhesive for use *in vivo* using the dECM material as the substrate described in the first aspect. The method includes the following steps:

Mixing a tissue active material, a base material, a thickener, a plasticizer and deionized water according to formulation amounts to obtain a pressure-sensitive adhesive slurry, coating the pressure-sensitive adhesive slurry on both sides of a biological substrate layer and drying the pressure-sensitive adhesive slurry to obtain the degradable pressure-sensitive adhesive for use *in vivo* using the dECM material as the substrate.

In the present application, a humectant and an antioxidant may also be added before the mixing.

In the present application, a specific process of the mixing is as follows: performing single mixing on the thickener and the humectant according to the formulation amounts, adding the deionized water, stirring the mixture at room temperature for 1 h, slowly adding the tissue active material, continuing stirring the mixture for 1 h, adding the plasticizer and the base material heated to 65-75°C, continuing stirring the mixture for 1 h, and finally adding the antioxidant and stirring the mixture at room temperature for 1 h.

Preferably, the drying is performed at a temperature of 20-50°C, which may be, for example, 20°C, 30°C, 40°C or 50°C; the drying is performed for 4-24 h, which may be, for example, 4 h, 8 h, 12 h, 14 h, 18 h, 20 h or 24 h.

In the present application, a specific process of coating the pressure-sensitive adhesive on the two sides of the biological substrate layer and drying the pressure-sensitive adhesive to obtain the degradable pressure-sensitive adhesive is as follows: coating the pressure-sensitive adhesive on both sides of the biological substrate layer, drying the pressure-sensitive adhesive for 4-24 h at 20-50°C, and covering the pressure-sensitive adhesive with release paper for later use.

Preferably, a pressure-sensitive adhesive layer formed after the drying has a thickness of 0.2-2 mm, which may be, for example, 0.2 mm, 0.5 mm, 0.8 mm, 1 mm, 1.2 mm, 1.5 mm, 1.8 mm or 2 mm.

In the present application, the in vivo degradable pressure-sensitive adhesive provided in the present application can be applied to uses such as the closure of wounds, tissue repair, the reinforcement of tissues and the healing of wounds *in vivo* and has excellent biocompatibility, and body reactions such as rejection and inflammation do not occur after the in vivo degradable pressure-sensitive adhesive is applied. In addition, the pressure-sensitive adhesive for the use *in vivo* provided in the present application can restore the integrity of tissues in a quick, site-directed and non-invasive manner and provide support for tissue remodeling and healing, thereby significantly improving the healing of surgical wounds.

Compared with the prior art, the present application has the following beneficial effects.

Materials such as paper, cellulose thin-films or polylactic acid-based materials are commonly used as substrates in existing degradable pressure-sensitive adhesives. However, this type of pressure-sensitive adhesive can be generally degraded only in external (*in vitro)* environment only and cannot complete the degradation process within the complex human body. In comparison, the degradable pressure-sensitive adhesive for use *in vivo* using the dECM material as the substrate provided in the present application is fully degradable under the action of *in vivo* enzymes. The dECM material and dECM gel ingredients can both be degraded *in vivo*, and all other components in the degradable pressure-sensitive adhesive for use *in vivo* provided in the present application can be degraded *in vivo*.

In addition, the tissue active material-dECM gel is added to the degradable pressure-sensitive adhesive for use *in vivo* using the dECM material as the substrate provided in the present application. Derived from enzymatically digested dECM, the dECM gel is rich in collagen and bioactive factors, such as fibroblast growth factors (FGFs), epidermal growth factors (EGFs) and vascular endothelial growth factors (VEGFs).These growth factors regulate cell proliferation, differentiation and migration, and promote the regeneration and repair of tissues. Compared with the dECM, the matrix gel obtained after the digestion has a lower molecular weight, which facilitates its absorption by the body. Therefore, the matrix gel obtained after the digestion can promote the healing of wounds more effectively at wound sites.

The present application provides a degradable pressure-sensitive adhesive for use *in vivo*. On the one hand, the biomaterial is used as the substrate layer, plays a role in structural support and has excellent biocompatibility as well as sufficient tensile strength and tear resistance. Therefore, this ensures that the pressure-sensitive adhesive is not prone to break or damage during use and the body reactions such as rejection and inflammation do not occur after the biomaterial is applied. The degradation products of some degradable materials synthesized after the production processes of physical or chemical modification disclosed in the existing art, such as the bioadhesive using caprolactone, glycolide or lactide as the substrate, are prone to the problems such as the inflammatory responses after the concentrated release. On the other hand, the adhesive of the in vivo degradable pressure-sensitive adhesive is obtained without complex chemical modification and process synthesis, and the biologically derived tissue active ingredient is added to some of the pressure-sensitive adhesive, which not only provides the pressure-sensitive adhesive good fluidity and water retention, but also promotes the healing of wounds and the repair of defective tissues. Moreover, other components of the pressure-sensitive adhesive are also degradable raw materials *in vivo* with excellent biocompatibility and high safety for the use *in vivo*, thereby avoiding the problems such as inflammatory responses caused by adhesives prepared through means such as chemical synthesis and modification.

The viscosity of the degradable pressure-sensitive adhesive for use *in vivo* provided in the present application can be adjusted to meet adhesion requirements of various scenarios. For example, for scenarios such as hemostasis, sealing and reducing body fluid exchange and infectious contamination between regions, a degradable medical pressure-sensitive adhesive with a relatively large viscosity can be applied, while for some scenarios that only need temporary bonding, such as an application scenario of transferring patch materials *in vitro* to tissues and organs, a degradable medical pressure-sensitive adhesive with a relatively small viscosity is applied. In addition, the above pressure-sensitive adhesive can be completely degraded after the implantation into the body, no foreign residue remains, and no encapsulation tissue or hardened tissue is formed. In particular, when the image detection is performed again after a cancerous tissue is removed, no shadow structure is generated. Optionally, a pharmaceutical ingredient may also be added to the component of the pressure-sensitive adhesive. The pressure-sensitive adhesive is attached to the skin or the tissue during application, and the drug is gradually released with the occurrence of a degradation reaction to implement a function of drug delivery.

### BRIEF DESCRIPTION OF DRAWINGS

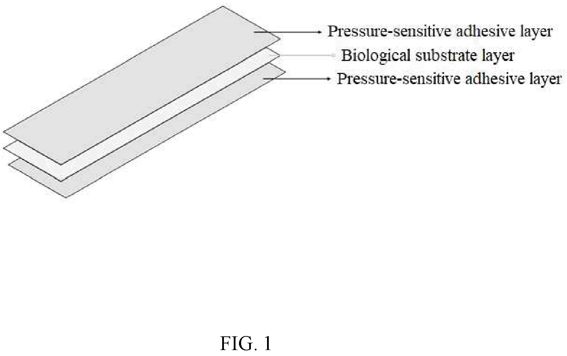

FIG. 1 is a structure diagram of a degradable pressure-sensitive adhesive for a use *in vivo* according to Example 1 of the present application.

### DETAILED DESCRIPTION

The technical solutions of the present application are further described below through specific examples in conjunction with the drawings. It is to be understood by those skilled in the art that these examples are provided to facilitate understanding of the present application and should not to be construed as limiting the present application.

Raw material compositions of pressure-sensitive adhesive layers of degradable pressure-sensitive adhesives for uses *in vivo* provided in examples and comparative examples of the present application are listed in Table 1.

**Table 1**

| | Raw Material Composition of Pressure-sensitive Adhesive Layer |
|---|---|
| Example 1 | polyvinyl alcohol, dECM gel, maltitol, trehalose, vitamin E, sorbitol and deionized water |
| Example 2 | polyvinyl alcohol, dECM gel, arabic gum, trehalose, vitamin E, sorbitol and deionized water |
| Example 3 | sodium carboxymethyl cellulose, dECM gel, maltitol, trehalose, vitamin E, sorbitol and deionized water |
| Example 4 | sodium carboxymethyl cellulose, dECM gel, arabic gum, trehalose, vitamin E, sorbitol and deionized water |
| Example 5 | sodium carboxymethyl cellulose, dECM gel, arabic gum, trehalose, vitamin E, glycerol and deionized water |
| Example 6 | sodium carboxymethyl cellulose, dECM gel, trehalose, vitamin E, glycerol and deionized water |
| Example 7 | polyvinyl alcohol, dECM gel, trehalose, vitamin E, sorbitol and deionized water |
| Example 8 | sodium carboxymethyl cellulose, dECM gel, arabic gum, trehalose, vitamin E (2.5%), glycerol and deionized water |
| Example 9 | sodium carboxymethyl cellulose, dECM gel, arabic gum, trehalose, vitamin E (5.2%), glycerol and deionized water |
| Example 10 | sodium carboxymethyl cellulose, dECM gel (10%), arabic gum, trehalose, vitamin E, glycerol and deionized water |
| Example 11 | sodium carboxymethyl cellulose, dECM gel (16%), arabic gum, trehalose, vitamin E, glycerol and deionized water |
| Example 12 | sodium carboxymethyl cellulose, dECM gel, arabic gum (20%), trehalose, vitamin E, glycerol and deionized water |
| Example 13 | sodium carboxymethyl cellulose, dECM gel, arabic gum (30%), trehalose, vitamin E, glycerol and deionized water |
| Example 14 | sodium carboxymethyl cellulose (8%), dECM gel, arabic gum, trehalose, vitamin E, glycerol and deionized water |
| Example 15 | sodium carboxymethyl cellulose (15%), dECM gel, arabic gum, trehalose, vitamin E, glycerol and deionized water |
| Comparative Example 1 | sodium carboxymethyl cellulose, dECM gel, arabic gum (5%), trehalose, vitamin E, glycerol and deionized water |
| Comparative Example 2 | sodium carboxymethyl cellulose, dECM gel, arabic gum (40%), trehalose, vitamin E, glycerol and deionized water |
| Comparative Example 3 | sodium carboxymethyl cellulose (2%), dECM gel, arabic gum, trehalose, vitamin E, glycerol and deionized water |
| Comparative Example 4 | sodium carboxymethyl cellulose (30%), dECM gel, arabic gum, trehalose, vitamin E, glycerol and deionized water |
| Comparative Example 5 | dECM gel, arabic gum, trehalose, vitamin E, glycerol and deionized water |
| Comparative Example 6 | sodium carboxymethyl cellulose, dECM gel (3%), arabic gum, trehalose, vitamin E, glycerol and deionized water |
| Comparative Example 7 | sodium carboxymethyl cellulose, dECM gel (30%), arabic gum, trehalose, vitamin E, glycerol and deionized water |
| Comparative Example 8 | sodium carboxymethyl cellulose, arabic gum, trehalose, vitamin E, glycerol and deionized water |
| Comparative Example 9 | sodium carboxymethyl cellulose, dECM gel, arabic gum, trehalose, vitamin E (1%), glycerol and deionized water |
| Comparative Example 10 | sodium carboxymethyl cellulose, dECM gel, arabic gum, trehalose, vitamin E (10%), glycerol and deionized water |
| Comparative Example 11 | sodium carboxymethyl cellulose, dECM gel, arabic gum, trehalose, glycerol and deionized water |

### Example 1

This example provides a degradable pressure-sensitive adhesive for use *in vivo* using a dECM material as a substrate. As shown in FIG. 1, the degradable pressure-sensitive adhesive comprises a decellularized porcine small intestinal submucosal (SIS) substrate layer and a pressure-sensitive adhesive layer disposed on each side of it.

The SIS is subjected to virus inactivation, decellularization, lyophilization, defatting and secondary lyophilization to obtain the substrate layer. The SIS is derived from boars each weighing approximately 150 kilograms which are subjected to closed feeding with plant-derived feed. The small intestine must be harvested within half an hour after the death of the boars, and the mucosa, muscularis and serosa of the small intestine must be removed within 12 h, leaving only the submucosa of the small intestine.

Based on a total mass of 100% for a raw material of the pressure-sensitive adhesive layer, the raw material of the pressure-sensitive adhesive layer includes a decellularized porcine small intestinal submucosa matrix gel with a mass percentage content of 14%, a polyvinyl alcohol base material with a mass percentage content of 12%, a maltitol thickener with a mass percentage content of 24%, a trehalose humectant with a mass percentage content of 5%, a vitamin E antioxidant with a mass percentage content of 3.5%, a sorbitol plasticizer with a mass percentage content of 18% and deionized water as a remainder.

The present application provides a method for preparing the above SIS substrate. The method includes the steps below.
(1) Virus inactivation: the treated SIS material was soaked in a 0.5% PAA solution for 30 min, and the residual PAA solution was washed with purified water.
(2) Decellularization: the SIS material was washed with shaking by using a mixed solution of 0.1% trypsin and 0.1% SDS for 2 h, and the residual solvent was washed with purified water.
(3) Lyophilization: the dECM material was lyophilized.
(4) Defatting: the lyophilized material was placed in a defatting reaction kettle containing a diethyl ether solution, the device operated for 16 h, the sample was taken and desorbed in a fume hood for 2 h, and the residual diethyl ether solution was washed with purified water.
(5) Secondary lyophilization: the defatted matrix material was placed into the lyophilizer again for lyophilization until the defatted matrix material was dried to obtain the single-layer SIS matrix.

This example also provides a method for preparing the above degradable pressure-sensitive adhesive. The method includes the steps below.

### (1) Preparation of the required raw materials and reagents

① 40 mg/mL SIS matrix gel: the SIS matrix was pulverized into a powder with a particle diameter of 250 µm, 2 g of the SIS matrix powder and 200 mg of pepsin (activity: 3450 U/mg, Sigma P6887-5G, derived from porcine gastric mucosa) were dissolved in 25 mL of HCl with a concentration of 0.01 mol/L and digested for 40 h, 2.8 mL of a 10-fold concentration PBS solution, 2.5 mL of a NaOH solution with a concentration of 0.01 mol/L and 19.7 mL of a 1-fold concentration PBS solution were added to the digested solution, and the mixture was stirred uniformly and set aside for later use. ② Polyvinyl alcohol: polyvinyl alcohol was dissolved in deionized water to prepare a solution at a concentration of 0.5 g/mL, and the solution was heated to 90°C until polyvinyl alcohol was completely dissolved.

### (2) Mixing in proportion

40 g of maltitol was weighed, trehalose, sorbitol and vitamin E were weighed according to the content proportions of the components, and the SIS matrix gel, the polyvinyl alcohol solution and deionized water were weighed according to the content proportions of the components. The maltitol, the trehalose and the deionized water were mixed and stirred for 1 h at room temperature, the SIS matrix gel was slowly added and continued to be stirred for 1 h, the sorbitol and the polyvinyl alcohol solution were added and continued to be stirred for 1 h, and finally, the vitamin E was added and stirred for 1 h at room temperature to obtain a pressure-sensitive adhesive.

### (3) Coating of the pressure-sensitive adhesive on a biomaterial substrate

The pressure-sensitive adhesive was coated onto one side of the SIS substrate with a thickness of 50 µm and a coating thickness of 500 µm. After drying at 26°C for 5 h, a release paper was applied, and the pressure-sensitive adhesive continued to be coated onto the other side of the SIS substrate, dried for 5 h at 26°C and covered with release paper to obtain the degradable pressure-sensitive adhesive.

### (4) Packaging and sterilization

The prepared degradable pressure-sensitive adhesive was placed in an aluminum foil bag for packaging and sterilized at 25 kGy.

### Example 2

This example provides an in vivo degradable pressure-sensitive adhesive thatuses a dECM material as its substrate. The degradable pressure-sensitive adhesive comprises a SIS substrate layer, with a pressure-sensitive adhesive layer disposed on each side of it.

The porcine small intestinal submucosa is subjected to virus inactivation, decellularization, lyophilization, defatting and secondary lyophilization to obtain the substrate layer. The porcine small intestinal submucosa is derived from boars, each weighing approximately 150 kilograms, that have been raised on a plant-based diet in a controlled environment. The small intestine must be taken within half an hour post-mortem. Subsequently, the mucosa, muscularis, and serosa must be removed from the intestine within 12 hours, leaving only the submucosa for further processing.

Based on a total mass of 100% for the pressure-sensitive adhesive layer formulation, the raw material includes a SIS matrix gel with a mass percentage content of 14%, a polyvinyl alcohol base material with a mass percentage content of 12%, an arabic gum thickener with a mass percentage content of 24%, a trehalose humectant with a mass percentage content of 5%, a vitamin E antioxidant with a mass percentage content of 3.5%, a sorbitol plasticizer with a mass percentage content of 18% and deionized water as a remainder.

The present application provides a method for preparing the above SIS substrate. The method includes the steps below.
(1) Virus inactivation: the treated SIS material was immersed in a 0.5% polyacrylic acid (PAA) solution for 30 min, and the residual PAA solution was washed with purified water.
(2) Decellularization: the SIS material was washed with shaking by using a mixed solution of 0.1% trypsin and 0.1% sodium dodecyl sulfate (SDS) for 2 h, and the residual solvent was washed with purified water.
(3) Lyophilization: the dECM material was lyophilized.
(4) Defatting: the lyophilized material was placed in a defatting reaction vessel containing a diethyl ether solution, the device operated for 16 h, the sample was then taken and desorbed in a fume hood for 2 h, and the residual diethyl ether solution was washed with purified water.
(5) Secondary lyophilization: the defatted matrix material was placed into the lyophilizer again for lyophilization until the defatted matrix material was dried to obtain the single-layer SIS matrix.

This example also provides a method for preparing the above in vivo degradable pressure-sensitive adhesive. The method includes the steps below.

### (1) Preparation of the required raw materials and reagents

① 40 mg/mL SIS matrix gel: the SIS matrix was pulverized into a powder with a particle diameter of 250 µm, 2 g of the SIS matrix powder and 200 mg of pepsin (activity: 3450 U/mg, Sigma P6887-5G, derived from porcine gastric mucosa) were dissolved in 25 mL of HCl with a concentration of 0.01 mol/L and digested for 40 h, 2.8 mL of a 10 × PBS solution, 2.5 mL of a NaOH solution with a concentration of 0.01 mol/L and 19.7 mL of a 1 × PBS solution were added to the digested solution, and the mixture was stirred uniformly and set aside for later use. ② Polyvinyl alcohol: polyvinyl alcohol was dissolved in deionized water to prepare a solution with a concentration range of 0.5 g/mL, and the solution was then heated to 90°C until polyvinyl alcohol was completely dissolved.

### (2) Mixing in proportion

40 g of arabic gum was weighed, trehalose, sorbitol and vitamin E were weighed according to the content proportions of the components, and the SIS matrix gel, the polyvinyl alcohol solution and deionized water were weighed according to the content proportions of the components. The arabic gum, the trehalose and the deionized water were mixed and stirred for 1 h at room temperature, the SIS matrix gel was slowly added and continued to be stirred for 1 h, the sorbitol and the polyvinyl alcohol solution were added and continued to be stirred for 1 h, and finally, the vitamin E was added and stirred for 1 h at room temperature to obtain a pressure-sensitive adhesive.

### (3) Coating of the pressure-sensitive adhesive on a biomaterial substrate

The pressure-sensitive adhesive was coated onto one side of the SIS substrate with a thickness of 50 µm and a coating thickness of 500 µm. After drying at 26°C for 5 h, a release paper was applied, and the pressure-sensitive adhesive continued to be coated onto the other side of the SIS substrate, dried for 5 h at 26°C and covered with release paper to obtain the degradable pressure-sensitive adhesive.

### (4) Packaging and sterilization

The prepared degradable pressure-sensitive adhesive was placed in an aluminum foil bag for packaging and sterilized at 25 kGy.

### Example 3

This example provides an in vivo degradable pressure-sensitive adhesive thatuses a dECM material as its substrate. The degradable pressure-sensitive adhesive comprises a SIS substrate layer, with a pressure-sensitive adhesive layer disposed on each side of it.

The porcine small intestinal submucosa is subjected to virus inactivation, decellularization, lyophilization, defatting and secondary lyophilization to obtain the substrate layer. The porcine small intestinal submucosa is derived from boars, each weighing approximately 150 kilograms, that have been raised on a plant-based diet in a controlled environment. The small intestine must be taken within half an hour post-mortem. Subsequently, the mucosa, muscularis, and serosa must be removed from the intestine within 12 hours, leaving only the submucosa for further processing.

Based on a total mass of 100% for the pressure-sensitive adhesive layer formulation, the raw material includes a SIS matrix gel with a mass percentage content of 14%, a sodium carboxymethyl cellulose base material (degree of substitution: 0.86, viscosity: 800 mpa·s) with a mass percentage content of 12%, a maltitol thickener with a mass percentage content of 24%, a trehalose humectant with a mass percentage content of 5%, a vitamin E antioxidant with a mass percentage content of 3.5%, a sorbitol plasticizer with a mass percentage content of 18% and deionized water as a remainder.

The present application provides a method for preparing the above SIS substrate. The method includes the steps below.
(1) Virus inactivation: the treated SIS material was immersed in a 0.5% polyacrylic acid (PAA) solution for 30 min, and the residual PAA solution was washed with purified water.
(2) Decellularization: the SIS material was washed with shaking by using a mixed solution of 0.1% trypsin and 0.1% SDS for 2 h, and the residual solvent was washed with purified water.
(3) Lyophilization: the dECM material was lyophilized.
(4) Defatting: the lyophilized material was placed in a defatting reaction vessel containing a diethyl ether solution, the device operated for 16 h, the sample was then taken and desorbed in a fume hood for 2 h, and the residual diethyl ether solution was washed with purified water.
(5) Secondary lyophilization: the defatted matrix material was placed into the lyophilizer again for lyophilization until the defatted matrix material was dried to obtain the single-layer SIS matrix.

This example also provides a method for preparing the above degradable pressure-sensitive adhesive. The method includes the steps below.

### (1) Preparation of the required raw materials and reagents

① 40 mg/mL SIS matrix gel: the SIS matrix was pulverized into a powder with a particle diameter of 250 µm, 2 g of the SIS matrix powder and 200 mg of pepsin (activity: 3450 U/mg, Sigma P6887-5G, derived from porcine gastric mucosa) were dissolved in 25 mL of HCl with a concentration of 0.01 mol/L and digested for 40 h, 2.8 mL of a 10 × PBS solution, 2.5 mL of a NaOH solution with a concentration of 0.01 mol/L and 19.7 mL of a 1 × PBS solution were added to the digested solution, and the mixture was stirred uniformly and set aside for later use.

### (2) Mixing in proportion

40 g of maltitol was weighed, trehalose, sodium carboxymethyl cellulose, sorbitol and vitamin E were weighed according to the content proportions of the components, and the SIS matrix gel and deionized water were weighed according to the content proportions of the components. The maltitol, the trehalose and the deionized water were mixed and stirred for 1 h at room temperature, the SIS matrix gel was slowly added and continued to be stirred for 1 h, the sorbitol and the sodium carboxymethyl cellulose were added and continued to be stirred for 1 h, and finally, the vitamin E was added and stirred for 1 h at room temperature to obtain a pressure-sensitive adhesive.

### (3) Coating of the pressure-sensitive adhesive on a biomaterial substrate

The pressure-sensitive adhesive was coated onto one side of the SIS substrate with a thickness of 50 µm and a coating thickness of 500 µm. After drying at 26°C for 5 h, a release paper was applied, and the pressure-sensitive adhesive continued to be coated onto the other side of the SIS substrate, dried for 5 h at 26°C and covered with release paper to obtain the degradable pressure-sensitive adhesive.

### (4) Packaging and sterilization

The prepared degradable pressure-sensitive adhesive was placed in an aluminum foil bag for packaging and sterilized at 25 kGy.

### Example 4

This example provides an in vivo degradable pressure-sensitive adhesive thatuses a dECM material as its substrate. The degradable pressure-sensitive adhesive comprises a SIS substrate layer, with a pressure-sensitive adhesive layer disposed on each side of it.

The porcine small intestinal submucosa is subjected to virus inactivation, decellularization, lyophilization, defatting and secondary lyophilization to obtain the substrate layer. The porcine small intestinal submucosa is derived from boars, each weighing approximately 150 kilograms, that have been raised on a plant-based diet in a controlled environment. The small intestine must be taken within half an hour post-mortem. Subsequently, the mucosa, muscularis, and serosa must be removed from the intestine within 12 hours, leaving only the submucosa for further processing.

Based on a total mass of 100% for the pressure-sensitive adhesive layer formulation, the raw material includes a SIS matrix gel with a mass percentage content of 14%, a sodium carboxymethyl cellulose base material (degree of substitution: 0.86, viscosity: 800 mpa·s) with a mass percentage content of 12%, an arabic gum thickener with a mass percentage content of 24%, a trehalose humectant with a mass percentage content of 5%, a vitamin E antioxidant with a mass percentage content of 3.5%, a sorbitol plasticizer with a mass percentage content of 18% and deionized water as a remainder.

The present application provides a method for preparing the above SIS substrate. The method includes the steps below.
(1) Virus inactivation: the treated SIS material was immersed in a 0.5% polyacrylic acid (PAA) solution for 30 min, and the residual PAA solution was washed with purified water.
(2) Decellularization: the SIS material was washed with shaking by using a mixed solution of 0.1% trypsin and 0.1% SDS for 2 h, and the residual solvent was washed with purified water.
(3) Lyophilization: the dECM material was lyophilized.
(4) Defatting: the lyophilized material was placed in a defatting reaction vessel containing a diethyl ether solution, the device operated for 16 h, the sample was then taken and desorbed in a fume hood for 2 h, and the residual diethyl ether solution was washed with purified water.
(5) Secondary lyophilization: the defatted matrix material was placed into the lyophilizer again for lyophilization until the defatted matrix material was dried to obtain the single-layer SIS matrix.

This example also provides a method for preparing the above degradable pressure-sensitive adhesive. The method includes the steps below.

### (1) Preparation of the required raw materials and reagents

① 40 mg/mL SIS matrix gel: the SIS matrix was pulverized into a powder with a particle diameter of 250 µm, 2 g of the SIS matrix powder and 200 mg of pepsin (activity: 3450 U/mg, Sigma P6887-5G, derived from porcine gastric mucosa) were dissolved in 25 mL of HCl with a concentration of 0.01 mol/L and digested for 40 h, 2.8 mL of a 10 × PBS solution, 2.5 mL of a NaOH solution with a concentration of 0.01 mol/L and 19.7 mL of a 1 × PBS solution were added to the digested solution, and the mixture was stirred uniformly and set aside for later use.

### (2) Mixing in proportion

40 g of arabic gum was weighed, trehalose, sodium carboxymethyl cellulose, sorbitol and vitamin E were weighed according to the content proportions of the components, and the SIS matrix gel and deionized water were weighed according to the content proportions of the components. The arabic gum, the trehalose and the deionized water were mixed and stirred for 1 h at room temperature, the SIS matrix gel was slowly added and continued to be stirred for 1 h, the sorbitol and the sodium carboxymethyl cellulose were added and continued to be stirred for 1 h, and finally, the vitamin E was added and stirred for 1 h at room temperature to obtain a pressure-sensitive adhesive.

### (3) Coating of the pressure-sensitive adhesive on a biomaterial substrate

The pressure-sensitive adhesive was coated on one side of the SIS substrate with a thickness of 50 µm and a coating thickness of 500 µm, dried for 5 h at 26°C and covered with release paper, and the pressure-sensitive adhesive continued to be coated on the other side of the SIS substrate, dried for 5 h at 26°C and covered with release paper to obtain the degradable pressure-sensitive adhesive.

### (4) Packaging and sterilization

The prepared degradable pressure-sensitive adhesive was placed in an aluminum foil bag for packaging and sterilized at 25 kGy.

### Example 5

This example provides an in vivo degradable pressure-sensitive adhesive thatuses a dECM material as its substrate. The degradable pressure-sensitive adhesive comprises a SIS substrate layer, with a pressure-sensitive adhesive layer disposed on each side of it.

The porcine small intestinal submucosa is subjected to virus inactivation, decellularization, lyophilization, defatting and secondary lyophilization to obtain the substrate layer. The porcine small intestinal submucosa is derived from boars, each weighing approximately 150 kilograms, that have been raised on a plant-based diet in a controlled environment. The small intestine must be taken within half an hour post-mortem. Subsequently, the mucosa, muscularis, and serosa must be removed from the intestine within 12 hours, leaving only the submucosa for further processing.

Based on a total mass of 100% for the pressure-sensitive adhesive layer formulation, the raw material includes a SIS matrix gel with a mass percentage content of 14%, a sodium carboxymethyl cellulose base material (degree of substitution: 0.86, viscosity: 800 mpa·s) with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder.

The present application provides a method for preparing the above SIS substrate. The method includes the steps below.
(1) Virus inactivation: the treated SIS material was immersed in a 0.5% polyacrylic acid (PAA) solution for 30 min, and the residual PAA solution was washed with purified water.
(2) Decellularization: the porcine small intestinal submucosal material was washed with shaking by using a mixed solution of 0.1% trypsin and 0.1% SDS for 2 h, and the residual solvent was washed with purified water.
(3) Lyophilization: the dECM material was lyophilized.
(4) Defatting: the lyophilized material was placed in a defatting reaction vessel containing a diethyl ether solution, the device operated for 16 h, the sample was then taken and desorbed in a fume hood for 2 h, and the residual diethyl ether solution was washed with purified water.
(5) Secondary lyophilization: the defatted matrix material was placed into the lyophilizer again for lyophilization until the defatted matrix material was dried to obtain the single-layer SIS matrix.

This example also provides a method for preparing the above degradable pressure-sensitive adhesive. The method includes the steps below.

### (1) Preparation of the required raw materials and reagents

① 40 mg/mL SIS matrix gel: the SIS matrix was pulverized into a powder with a particle diameter of 250 µm, 2 g of the SIS matrix powder and 200 mg of pepsin (activity: 3450 U/mg, Sigma P6887-5G, derived from porcine gastric mucosa) were dissolved in 25 mL of HCl with a concentration of 0.01 mol/L and digested for 40 h, 2.8 mL of a 10 X PBS solution, 2.5 mL of a NaOH solution with a concentration of 0.01 mol/L and 19.7 mL of a 1 × PBS solution were added to the digested solution, and the mixture was stirred uniformly and set aside for later use.

### (2) Mixing in proportion

40 g of arabic gum was weighed, trehalose, sodium carboxymethyl cellulose and vitamin E were weighed according to the content proportions of the components, and the SIS matrix gel, glycerol and deionized water were weighed according to the content proportions of the components. The arabic gum, the trehalose and the deionized water were mixed and stirred for 1 h at room temperature, the SIS matrix gel was slowly added and continued to be stirred for 1 h, the glycerol and the sodium carboxymethyl cellulose were added and continued to be stirred for 1 h, and finally, the vitamin E was added and stirred for 1 h at room temperature to obtain a pressure-sensitive adhesive.

### (3) Coating of the pressure-sensitive adhesive on a biomaterial substrate

The pressure-sensitive adhesive was coated on one side of the SIS substrate with a thickness of 50 µm and a coating thickness of 500 µm, dried for 5 h at 26°C and covered with release paper, and the pressure-sensitive adhesive continued to be coated on the other side of the SIS substrate, dried for 5 h at 26°C and covered with release paper to obtain the degradable pressure-sensitive adhesive.

### (4) Packaging and sterilization

The prepared degradable pressure-sensitive adhesive was placed in an aluminum foil bag for packaging and sterilized at 25 kGy.

### Example 6

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, sodium carboxymethyl cellulose with a mass percentage content of 36%, a trehalose humectant with a mass percentage content of 5%, a vitamin E antioxidant with a mass percentage content of 3.5%, a glycerol plasticizer with a mass percentage content of 18%, and deionized water as a remainder. That is, no arabic gum was added to the adhesive system as the thickener, but the sodium carboxymethyl cellulose was used as both the base material and the thickener. The rest was the same as that in Example 5.

### Example 7

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a polyvinyl alcohol base material with a mass percentage content of 12%, a trehalose humectant with a mass percentage content of 5%, a vitamin E antioxidant with a mass percentage content of 3.5%, sorbitol with a mass percentage content of 42%, and deionized water as a remainder. That is, no maltitol thickener was added to the adhesive system, but the sorbitol was used as both the plasticizer and the thickener. The rest was the same as that in Example 1.

### Example 8

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 2.5%, a glycerol plasticizer with a mass percentage content of 18%, and deionized water as a remainder. The rest was the same as that in Example 5.

### Example 9

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 5.2%, a glycerol plasticizer with a mass percentage content of 18%, and deionized water as a remainder. The rest was the same as that in Example 5.

### Example 10

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 10%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18%, and deionized water as a remainder. The rest was the same as that in Example 5.

### Example 11

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 16%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18%, and deionized water as a remainder. The rest was the same as that in Example 5.

### Example 12

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 12%, an arabic gum thickener with a mass percentage content of 20%, a trehalose humectant with a mass percentage content of 5%, a vitamin E antioxidant with a mass percentage content of 3.5%, a glycerol plasticizer with a mass percentage content of 18%, and deionized water as a remainder. The rest was the same as that in Example 5.

### Example 13

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 12%, an arabic gum thickener with a mass percentage content of 30%, a trehalose humectant with a mass percentage content of 5%, a vitamin E antioxidant with a mass percentage content of 3.5%, a glycerol plasticizer with a mass percentage content of 18%, and deionized water as a remainder. The rest was the same as that in Example 5.

### Example 14

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 8%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18%, and deionized water as a remainder. The rest was the same as that in Example 5.

### Example 15

This example differs from Example 5 in that the composition of the pressure-sensitive adhesive layeris modified. Based on a total mass of 100% for the formulation,, the raw materials of the pressure-sensitive adhesive layer includes a SIS matrix gel with a mass percentage content of 14%, a sodium carboxymethyl cellulose base material with a mass percentage content of 15%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 1

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 12%, an arabic gum thickener with a mass percentage content of 5%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 2

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 12%, an arabic gum thickener with a mass percentage content of 40%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 3

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 2%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 4

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 30%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 5

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 6

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 3%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 7

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 30%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 8

This comparative example differs from Example 5 in that the composition the pressure-sensitive adhesive layer is modified. Based on a total mass of 100% for the formulation, the raw material of the pressure-sensitive adhesive layer includes a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The rest was the same as that in Example 5.

### Comparative Example 9

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 1%, a glycerol plasticizer with a mass percentage content of 18% and deionized water as a remainder. The rest was the same as that in Example 5.

### Comparative Example 10

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 10%, a glycerol plasticizer with a mass percentage content of 18% and deionized water as a remainder. The rest was the same as that in Example 5.

### Comparative Example 11

This comparative example differs from Example 5 in that the composition the pressure-sensitive adhesive layer is modified. Based on a total mass of 100% for the formulation, the raw material of the pressure-sensitive adhesive layer includes a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5% and a glycerol plasticizer with a mass percentage content of 18% and deionized water as a remainder. The rest was the same as that in Example 5.

### Test conditions

The in vivo degradable pressure-sensitive adhesives provided in Examples 1 to 15 and Comparative Examples 1 to 11 were packaged and sterilized for the test. The test methods are as follows.
(1) Initial tack: the test was tested according to the GB/T 4852-2002, the experimental result is represented by the number of the largest steel ball to which the pressure-sensitive adhesive tape can adhere within a specified range, three parallel samples in each group were tested, and an average value was calculated.
(2) Holding power: the test was performed according to the GB 4851-1984 standard, three parallel samples in each group were tested, and an average value was taken.
(3) 180° peel strength: the test was performed according to the GB/T 2792-1998 standard, three parallel samples in each group were tested, and an average value was taken.
(4) Discoloration: the packaged and sterilized pressure-sensitive adhesive was placed in a test chamber at 50±2°C for 5 days, the degree of yellowing of the pressure-sensitive adhesive was observed, and the degree of discoloration was evaluated according to the ASTM D1925 standard shown in Table 2 within 24 h.

**Table 2 Classification grades of discoloration**

| | |
|---|---|
| Grade 0 | no visible discoloration |
| Grade 1 | extremely slight discoloration |
| Grade 2 | slight discoloration |
| Grade 3 | apparent discoloration |
| Grade 4 | severe discoloration |

(5) Elongation at break: the test was performed according to the GB 6329-1986 standard, three parallel samples in each group were tested, and an average value was taken.
(6) Wound healing rate of mouse: wound models of mice were established, and six- to eight-week-old mice (the mice were purchased from a commercially available experimental animal company with a relevant qualification) were prepared; the mice were shaved on the back, and the skin and the subcutaneous membrane in the dorsal area between the base of the neck and the shoulder were excised to form a wound with a diameter of 1 cm, and a circular ring-shaped silicone splint membrane was sutured with a 6-0 nylon thread to prevent the wound from shrinking; to observe differences in the wound healing of the mice, the wound was covered with transparent tracing paper on Days 3, 7, 10, 12 and 15 after the wound, a line was drawn along an outer edge of the wound, the wound area was measured, and "wound healing rate = (1-incision area/initial incision area) × 100%" was calculated.
(7) pH value test: the test was performed according to the GB/T 14518-1993 standard, three parallel samples in each group were tested, and an average value was taken.
(8) Cytotoxicity test: the measurement was performed using an MTT method according to GB/T 16886.5-2017, "Biological evaluation of medical devices-Part 5: Tests for in vitro cytotoxicity".
(9) Pyrogen test: potential pyrogen reactions of the samples were evaluated through a rabbit pyrogen test according to Chinese Pharmacopoeia (2020), Volume IV: 1142. Pyrogen test method.

The test results are shown in Table 3.

**Table 3**

| | Initial Viscosity (mm) | Holding Power (s) | 180° Peel Strength (N·cm⁻¹) | Discoloration Grade |
|---|---|---|---|---|
| Example 1 | 4.762 | 151 | 2.5 | grade 1 |
| Example 2 | 4.762 | 146 | 3.1 | grade 1 |
| Example 3 | 5.556 | 265 | 3.5 | grade 1 |
| Example 4 | 6.350 | 273 | 3.9 | grade 1 |
| Example 5 | 10.319 | 325 | 9.6 | grade 0 |
| Example 6 | 3.969 | 102 | 2.0 | grade 0 |
| Example 7 | 2.381 | 46 | 0.9 | grade 1 |
| Example 8 | 10.054 | 348 | 10.2 | grade 1 |
| Example 9 | 9.750 | 322 | 8.7 | grade 0 |
| Example 10 | 10.054 | 294 | 8.2 | grade 1 |
| Example 11 | 10.319 | 251 | 9.8 | grade 1 |
| Example 12 | 9.525 | 276 | 8.1 | grade 1 |
| Example 13 | 11.112 | 211 | 7.6 | grade 2 |
| Example 14 | 8.731 | 233 | 5.2 | grade 1 |
| Example 15 | 10.319 | 301 | 9.5 | grade 1 |
| Comparative Example 1 | 8.731 | 208 | 4.9 | grade 1 |
| Comparative Example 2 | 12.70 | 58 | 1.2 | grade 2 |
| Comparative Example 3 | 7.144 | 195 | 3.8 | grade 1 |
| Comparative Example 4 | 11.906 | 246 | 7.9 | grade 1 |
| Comparative Example 5 | 3.175 | 79 | 1.5 | grade 1 |
| Comparative Example 6 | 10.054 | 273 | 6.9 | grade 1 |
| Comparative Example 7 | 9.525 | 191 | 5.7 | grade 1 |
| Comparative Example 8 | 10.319 | 282 | 7.2 | grade 1 |
| Comparative Example 9 | 10.054 | 293 | 8.4 | grade 2 |
| Comparative Example 10 | 9.525 | 186 | 7.9 | grade 0 |
| Comparative Example 11 | 10.319 | 279 | 7.5 | grade 3 |

The following can be seen from Table 3:
Maltitol and arabic gum are used as the thickeners in Examples 1 and 2, respectively, and there is no significant difference in the initial tack, holding power and peel strength of the in vivo degradable pressure-sensitive adhesives prepared with these added ingredients. However, the incorporation of sodium carboxymethyl cellulose as the base material effectively improved the final viscosity characteristics of the product.

The formulation of Example 5 includes the addition of a SIS matrix gel with a mass percentage content of 14%, a sodium carboxymethyl cellulose base material with a mass percentage content of 12%, an arabic gum thickener with a mass percentage content of 24%, a trehalose humectant with a mass percentage content of 5%, a vitamin E antioxidant with a mass percentage content of 3.5%, a glycerol plasticizer with a mass percentage content of 18% and deionized water as a remainder. The prepared in vivo degradable pressure-sensitive adhesive has the best initial tack and holding power values, the best peel strength and the lowest discoloration grade. These also indicate that the in vivo degradable pressure-sensitive adhesive prepared in Example 5 exhibits the strongest adhesion during the connection to contact with surfaces and exhibits better stability during storage.

Carboxymethyl cellulose is used as both the base material and the thickener in Example 6, and the initial tack, holding power and peel strength of the in vivo degradable pressure-sensitive adhesive are significantly reduced compared to those in Examples 1 to 5. Sorbitol is used as both the thickener and the plasticizer in Example 7, and the initial tack, holding power and peel strength of the in vivo degradable pressure-sensitive adhesive are also significantly reduced compared to those in Examples 1 to 5.

Comparative Example 1 contains less thickener, and the initial tack and holding power of the pressure-sensitive adhesive are reduced, indicating that the thickener plays an important role in adjusting the viscosity of the in vivo degradable pressure-sensitive adhesive. However, when the content of the thickener is increased to 40%, the fluidity of the adhesive material on the surface of the in vivo degradable pressure-sensitive adhesive increases after the in vivo degradable pressure-sensitive adhesive is subjected to irradiation sterilization, and the holding power and the peeling strength exhibit cliff-like decreases. The reason is that the irradiation causes the glycosidic bond in the thickener to break and generate new groups, changing the chemical bonds, reactive groups and crystal structure of the thickener polysaccharide and resulting in a change in the overall viscosity of the in vivo degradable pressure-sensitive adhesive. The formulation of Comparative Example 3 contains less base material, and the initial tack and holding power of the pressure-sensitive adhesive are reduced. The formulation of Comparative Example 4 contains more base material. Although the initial tack, holding power and peel strength of the pressure-sensitive adhesive are high, during the actual coating process, the base material can easily curl during operation due to poor fluidity of the adhesive system, resulting in an uneven surface of the pressure-sensitive adhesive product. In Comparative Example 5, no base material ingredient is added, and the initial tack, holding power and peel strength of the pressure-sensitive adhesive are significantly reduced. It indicates that the base material plays an important role in the formulation of the product. In addition, comparing Examples 5 and 6 and Comparative Example 5, it can be seen that there is a synergistic effect between the thickener and the base material which are provided in the present application, and both the thickener and the base material are indispensable, otherwise the overall performance of the in vivo degradable pressure-sensitive adhesive is affected.

Comparative Example 6 contains less dECM gel, Comparative Example 7 contains more dECM gel, and Comparative Example 8 contains no dECM gel. The results show that when the dECM gel is added too much, the holding power of the pressure-sensitive adhesive decreases significantly. The reason is that the irradiation energy causes the water in the gel system to ionize and generate a large number of free radicals to react with proteins, resulting in irreversible crosslinking reactions between intermolecular amino and hydroxyl. In the process of wound repair, the addition of the dECM gel significantly promotes the wound healing rate (Table 4), indicating that the dECM gel plays an important role in the pressure-sensitive adhesive.

**Table 4 Wound healing rate of mouse (%)**

| Number of Days | Blank Group | Comparative Example 8 | Example 5 | Comparative Example 7 |
|---|---|---|---|---|
| 3 day | 9.27±0.24 | 9.76±0.51 | 11.83±1.24 | 13.62±1.63 |
| 7 day | 25±5.7 | 28±2.67 | 43±0.39 | 49±2.16 |
| 10 day | 54±1.28 | 52±3.18 | 64±2.46 | 72±0.99 |
| 12 day | 71±1.24 | 78±1.6 | 81±1.38 | 84±1.01 |
| 15 day | 90±0.38 | 91±1.79 | 96±0.17 | 98±0.47 |

Less vitamin E is added in Comparative Example 9, more vitamin E is added in Comparative Example 10, and no vitamin E is added in Comparative Example 11. Although the viscosity results show that the amount of vitamin E added has little effect on the viscosity, the yellowing test shows relatively high degrees of discoloration in Comparative Examples 9 and 11, indicating that the antioxidant vitamin E plays an important role in the pressure-sensitive adhesive.

**Table 5**

| | Elongation at Break (%) | pH Value Test | Cytotoxicity | Pyrogen Reaction |
|---|---|---|---|---|
| Example 4 | 27.28 | 7.62 | No potential cytotoxicity | The extraction solution was injected into the experimental rabbits through the ear veins, and the body temperatures of the rabbits did not rise above 0.6°C, all meeting the regulation of the pyrogen test. |

In Table 5, the elongation at break, pH, cytotoxicity and pyrogen reaction results of the in vivo degradable pressure-sensitive adhesive prepared from a SIS as the substrate where a SIS gel is coated on two sides as the active substance, sodium carboxymethyl cellulose as the base material, arabic gum as the thickener, trehalose as the humectant, vitamin E as the antioxidant and glycerol as the plasticizer are measured and all meet the requirements of national standards for use.

The applicant has stated that although the process methods in the present application are described through the above-mentioned examples, the present application is not limited to the above-mentioned processes and steps, which means that the implementation of the present application does not necessarily depend on the above-mentioned processes and steps. It should be apparent to those skilled in the art that any improvements made to the present application, equivalent replacements of raw materials selected in the present application and additions of adjuvant ingredients thereof, and selections of specific methods, etc., all fall within the protection scope and the disclosed scope of the present application.

## Claims

1. An in vivo degradable pressure-sensitive adhesive using a decellularized extracellular matrix (Decm) material as a substrate, comprising a biological substrate layer and a pressure-sensitive adhesive layer disposed on two sides of the biological substrate layer;
wherein based on a total mass of 100% for a raw material of the pressure-sensitive adhesive layer, the raw material of the pressure-sensitive adhesive layer comprises a base material with a mass percentage content of 5% to 25%, a tissue active material with a mass percentage content of 5% to 18%, a thickener with a mass percentage content of 10% to 35%, a plasticizer with a mass percentage content of 10% to 50% and deionized water with a mass percentage content of 10% to 60%.

2. The in vivo degradable pressure-sensitive adhesive according to claim 1, wherein based on a total mass of 100% for the pressure-sensitive adhesive layer, the raw material of the pressure-sensitive adhesive layer comprises a base material with a mass percentage content of 8% to 15%, a tissue active material with a mass percentage content of 10% to 16%, a thickener with a mass percentage content of 20% to 30%, a plasticizer with a mass percentage content of 15% to 20% and deionized water with a mass percentage content of 19% to 45%.

3. The in vivo degradable pressure-sensitive adhesive according to claim 1 or 2, wherein the tissue active material comprises any one of a skin, a pericardium, an amniotic membrane, a small intestinal submucosa or a bladder basement membrane of a mammal or a combination of at least two of dECM gels.

4. The in vivo degradable pressure-sensitive adhesive according to claim 1, wherein the base material comprises a degradable natural macromolecular compound and/or a degradable modified natural macromolecular compound;
the degradable natural macromolecular compound comprises any one or a combination of at least two of starch, dextrin, peach gum, arabic gum, bone glue, hide glue, gelatin, fish glue, shellac, vegetable protein or casein; and
the degradable modified natural macromolecular compound comprises any one or a combination of at least two of sodium carboxymethyl cellulose, modified starch or polyvinyl alcohol.

5. The in vivo degradable pressure-sensitive adhesive according to claim 1, wherein the thickener comprises any one or a combination of at least two of starch, modified starch, xanthan gum, maltitol, fructooligosaccharide, sorbitol, xylitol, lactitol, mannitol, erythritol, hydrogenated starch hydrolysate, gelatin, cellulose, methyl cellulose, hydroxyethyl cellulose, hydroxypropyl methylcellulose, sodium carboxymethyl cellulose, arabic gum, tamarind gum, sesbania gum, agar, sodium alginate, carrageenan, pectin or β-cyclodextrin.

6. The in vivo degradable pressure-sensitive adhesive according to claim 1, wherein the plasticizer comprises a polyol compound; and
the polyol compound comprises any one or a combination of at least two of glycerol, sorbitol or ethylene glycol.

7. The in vivo degradable pressure-sensitive adhesive according to any one of claims 4 to 6, wherein the base material, the thickener and the plasticizer are of different types.

8. The in vivo degradable pressure-sensitive adhesive according to any one of claims 4 to 6, wherein the base material comprises sodium carboxymethyl cellulose;
the thickener comprises arabic gum; and
the plasticizer comprises glycerol.

9. The in vivo degradable pressure-sensitive adhesive according to claim 1, wherein based on the total mass of 100% for the raw material of the pressure-sensitive adhesive layer, the raw material of the pressure-sensitive adhesive layer further comprises 0.1% to 21.6% of a humectant and 2.5% to 5.2% of an antioxidant;
the humectant comprises a polyol humectant and/or a sugar humectant; and
the antioxidant comprises any one or a combination of at least two of vitamin E, vitamin C, flavonoid, tea polyphenol, phytic acid, glucosamine, lactonic acid or sodium hyaluronate.

10. The in vivo degradable pressure-sensitive adhesive according to claim 1, wherein a material of the biological substrate layer comprises the dECM material; and
the dECM material comprises any one or a combination of at least two of a skin, a pericardium, an amniotic membrane, a small intestinal submucosa or a bladder basement membrane of a mammal.

11. A method for preparing the in vivo degradable pressure-sensitive adhesive using the dECM material as the substrate according to any one of claims 1 to 10, comprising the following steps:
mixing a tissue active material, a base material, a thickener, a plasticizer and deionized water according to formulation amounts to obtain a pressure-sensitive adhesive slurry, coating the pressure-sensitive adhesive slurry on two sides of a biological substrate layer and drying the pressure-sensitive adhesive slurry to obtain the in vivo degradable pressure-sensitive adhesive using the dECM material as the substrate.

12. The method according to claim 11, wherein the drying is performed at a temperature of 20-50°C for a period of 4-24 h.

13. The method according to claim 11, wherein a pressure-sensitive adhesive layer formed after the drying has a thickness of 0.2-2 mm.
